Europäisches Patentamt

(19) European Patent Office

Office européen des brevets

(11) Numéro de publication: **0 183 886**
**B1**

(12) **FASCICULE DE BREVET EUROPEEN**

(45) Date de publication du fascicule du brevet: **18.01.89**

(51) Int. Cl.⁴: **A 61 M 5/315**

(21) Numéro de dépôt: **84440068.9**

(22) Date de dépôt: **07.12.84**

(54) Seringue pour dentisterie.

(30) Priorité: **09.11.84 FR 8417226**

(43) Date de publication de la demande:
**11.06.86 Bulletin 86/24**

(45) Mention de la délivrance du brevet:
**18.01.89 Bulletin 89/03**

(84) Etats contractants désignés:
**AT BE CH DE GB IT LI LU NL SE**

(56) Documents cités:
**EP-A-0 109 913**
**AT-B- 212 625**
**CH-A- 202 975**
**GB-A- 108 917**
**US-A-3 517 668**

(73) Titulaire: **MICRO-MEGA**
**5-12 rue du Tunnel**
**F-25000 Besancon (FR)**

(72) Inventeur: **Leonard, Henri**
**4 rue Jean de vienne**
**F-25000 Besancon (FR)**
Inventeur: **Seigneurin, Michel**
**St Cergues**
**F-74140 Douvaine (FR)**

(74) Mandataire: **Poupon, Michel**
**3, rue Thiers B.P. 247**
**F-88007 Epinal Cédex (FR)**

Courier Press, Leamington Spa, England.

## Description

La présente invention a pour objet une seringue destinée à l'injection intra-ligamentaire de produits liquides, notamment de produits anesthésiques, du type se composant d'un corps dont la portion avant, sur laquelle est adaptée l'aiguille d'injection, sert de logement à un conteneur dudit produit liquide, constitué par un tube de verre dont l'extrémité arrière est obturée par un bouchon de caoutchouc ou en matériau analogue, tandis que la portion arrière du corps sert de guide à un piston actionné par une crémaillère, de manière telle que, lors de la manoeuvre de la seringue par l'opérateur, le piston progresse pas à pas vers l'avant en refoulant ledit bouchon vers l'avant dans l'intérieur du conteneur, et par suite le produit liquide dans l'aiguille d'injection, le piston se terminant, à son extrémité avant, par une tête d'un diamètre sensiblement égal en diamètre interne du conteneur.

Une seringue de ce type a fait l'objet du brevet US—A—3517668, bien que l'application à l'injection intraligamentaire de produits liquides ne soit pas spécifiée.

Or, on a constaté que quand la face avant du piston exerce sa pression sur la face arrière du bouchon, la matière de ce dernier manifeste une tendance à fluer entre la paroi de verre du conteneur et la périphérie du piston métallique. Ce laminage risque d'avoir plusieurs conséquences:
— un freinage du piston
— éventuellement des fuites du liquide anesthésique, qui peut lui-même devenir un élément destructeur du mécanisme,
— et surtout un bourrage, accroissant la pression sur le verre du conteneur, d'où un danger de rupture et même d'explosion du conteneur, notamment si le verre du conteneur est trop mince ou de qualité insatisfaisante.

Pour éliminer ces inconvénients selon la présente invention, on prévoit que la tête présente vers l'avant une concavité déterminant la migration vers l'axe du matériau de bouchon lors de l'avance du piston.

Ainsi, les efforts subis par le matériau de bouchon, au lieu d'être orientés vers l'extérieur contre la paroi de verre, sont orientés vers la creusure de la tête, où le matériau comprimé a tendance à s'épancher. On réduit donc encore les risques de rupture, tout en favorisant le glissement du bouchon vers l'avant.

Il a certes déjà été proposé par le brevet GB—A—108917 de réaliser une tête de piston qui présente vers l'avant une concavité, mais pour une finalité totalement différente.

On va maintenant commenter l'invention en se référant au dessin annexé sur lequel:

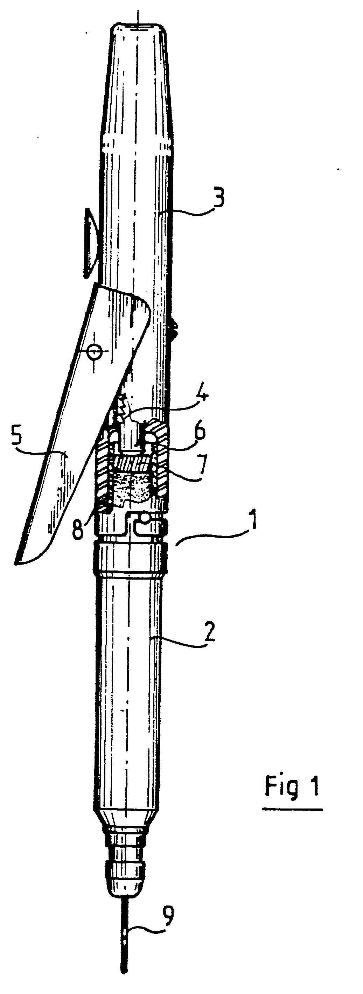

— la figure 1 est une vue d'ensemble en coupe partielle d'une seringue conforme à l'invention;
— la figure 2 est une vue fragmentaire à plus grande échelle de la figure 1;
— la figure 3 est une vue semblable à la figure 2, concernant une caractéristique particulière de l'invention et,

— la figure 4 est une vue semblable à la figure 3, concernant le perfectionnement selon l'invention.

Sur toutes les figures, les mêmes références désignent les mêmes éléments, à savoir:

Le corps 1 de la seringue se composant d'une portion avant 2 et d'une portion arrière 3, la crémaillère 4, quand elle est actionnée par la poignée 5, fait avancer le piston 6, qui vient refouler le bouchon de caoutchouc 7, obstruant l'arrière du conteneur 8, de manière à expulser le liquide anesthésique par l'aiguille 9.

Dans la réalisation connue, la tête du piston 6 en forme de cylindre à diamètre constant, sensiblement inférieur à celui du bouchon 7, se terminait par un chanfrein 10. Il en résultait pour le matériau du bouchon 7 une tendance à fluer, comme indiqué par les flèches F, vers l'espace 11 demeurant libre autour de la périphérie du piston 6.

Ce laminage du caoutchoux du bouchon 7 dans la zone 11 entraîne les inconvénients énumérés ci-dessus: freinage du déplacement du piston 6 vers la gauche, fuites possibles du liquide et surtout risques de rupture de l'embouchure de conteneur 8 sous l'effet de la pression radiale F'.

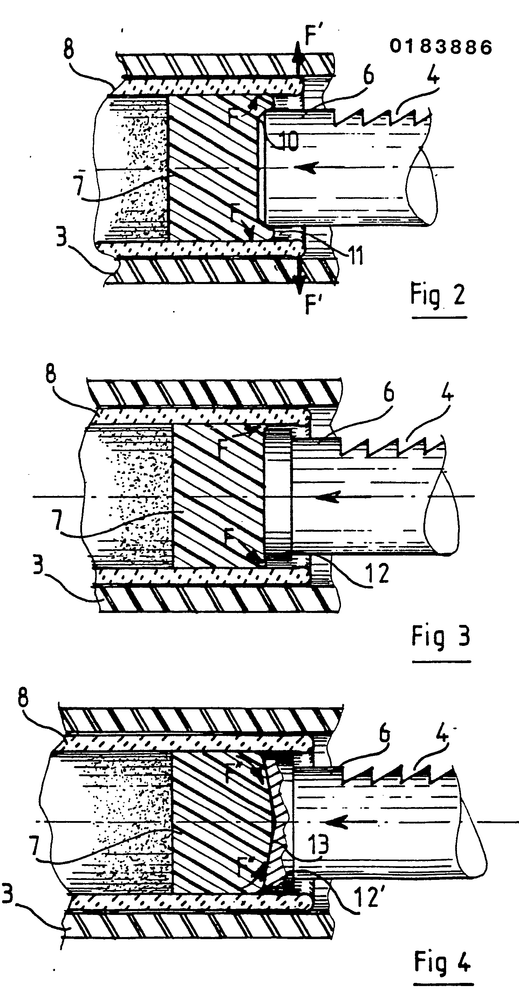

La figure 3 illustre la solution présentée par l'invention: l'extrémité avant du piston 6 se termine par une tête cylindrique 12 dont le diamètre est sensiblement supérieur à celui du piston 6 lui-même, pour approcher celui du bouchon 7. Le phénomène de fluage du caoutchouc entre le piston et la paroi du conteneur 8 est ainsi réduit au minimum, de sorte que les efforts F' deviennent négligeables, ce qui élimine les risques de rupture de conteneur.

La figure 4 illustre une variante de la figure 3 selon laquelle la tête 12' est creusée en 13 vers l'avant, de sorte que le matériau du bouchon 7 s'épanche ver l'axe suivant F''. De cette sorte, il n'y a plus fluage périphérique, donc ni freinage du piston ni risque d'éclatement de l'embouchure du conteneur.

## Revendication

Seringue destinée à l'injection intra-ligamentaire de produits liquides, notamment de produits anesthésiques, du type se composant d'un corps dont la portion avant, sur laquelle est adaptée l'aiguille d'injection, sert de logement à un conteneur dudit produit liquide, constitué par un tube de verre dont l'extrémité arrière est obturée par un bouchon (7) de caoutchouc on en matériau analogue, tandis que la portion arrière du corps sert de guide à un piston (6) actionné par une crémaillère (4), de manière telle que, lors de la manoeuvre de la seringue par l'opérateur, le piston progresse pas à pas vers l'avant en refoulant ledit bouchon vers l'avant dans l'intérieur du conteneur, et par suite le produit liquide dans l'aiguille d'injection, le piston (6) se terminant, à son extrémité avant, par une tête (12, 12') d'un diamètre sensiblement égal en diamètre interne du conteneur (8), caractérisé en ce que la tête présente vers l'avant une concavité déterminant la migration vers l'axe du matériau du bouchon (7) lors de l'avance du piston (6).

## EP 0 183 886 B1

**Patentanspruch**

Spritze für die intra-ligamentäre Injektion von flüssigen Produkten, insbesondere von anästhesierenden Produkten, mit einem Körper, dessen Vorderteil, an dem die Injektionsnadel angebracht ist, für einen Behälter des flüssigen Produktes als Aufnahme dient, der aus einem Glasrohr besteht, dessen hinteres Ende durch einen Stopfen (7) aus Gummi oder aus einem ähnlichen Material verschlossen ist, wohingegen das Hinteteil des Körpers einem über eine Zahnstange (4) betätigten Kolben (6) derart als Führung dient, daß während der Bewegung der Spritze durch den Bediener der Kolben, den Stophen nach vorne in das Innere des Behälters und als Folge das flüssige Produkt in die Injektionsnadel drückend, sich Schritt um Schritt nach vorne bewegt, wobei der Kolben (6) an seinem vorderen Ende in einen Kopf (12, 12') mit einem im wesentlichen gleichen Durchmesser wie der innere Durchmesser des Behälters (8) übergeht, dadurch gekennzeichnet, daß der Kopf nach vorne eine Aushöhlung aufweist, die die Migration zur Achse des Materials des Stopfens (7) während des Vorrückens des Kolbens (6) bewirkt.

**Claim**

Syringe intended for the ultra-ligamentary injection of liquid products, especially anaesthetic products, of the type composed of a body the forward portion of which, to which the injection needle is fitted, serves as housing for a container of the said liquid product, constituted by a glass tube the rear extremity of which is closed by a plug (7) of rubber or analogous material, while the rear portion of the body serves as guide for a piston (6) actuated by a rack (4), in such manner that when the operator operates the syringe, the piston progresses forward step by step, thrusting the said plug forward into the interior of the container, and consequently the liquid product into the injection needle, the piston (6) being terminated, at its forward extremity, by a head (12, 12') of a diameter substantially equal to the internal diameter of the container (8), characterised in that the head forwardly presents a concavity causing migration of the material of the plug (7) towards the axis in the advance of the piston (6).

Fig 1

0183886

Fig 2

Fig 3

Fig 4